# EUROPEAN PATENT SPECIFICATION

(11) **EP 3 804 674 B1**
(45) Date of publication and mention of the grant of the patent: **05.11.2025**
(21) Application number: 19815375.1
(22) Date of filing: 03.06.2019
(51) Int. Cl.: A61F 13/01, A61F 13/05, A61M 1/00, A61F 13/14

(54) **PROTECTIVE DRESSING FOR ABDOMINAL VISCERA IN NEGATIVE PRESSURE THERAPY**
SCHUTZVERBAND FÜR ABDOMINALE VISZERA IN DER UNTERDRUCKTHERAPIE
TIMBRE PROTECTEUR POUR LES VISCÈRES ABDOMINAUX DANS LA THÉRAPIE PAR PRESSION NÉGATIVE

(30) Priority: 06.06.2018 ES 201830853 U
(43) Date of publication of application: 14.04.2021
(73) Proprietor: Servicio Cántabro de Salud, 39011 Santander (Cantabria) (ES)
(72) Inventor: CASTILLO SUESCUN, Federico, 30011 SANTANDER (ES)
(74) Representative: Herrero & Asociados, S.L.
(86) International application number: PCT/ES2019/070372
(87) International publication number: WO 2019/234273

(56) References cited:
- CN-U- 208 876 987
- ES-A1- 2 221 555
- ES-T3- 2 553 768
- GB-A- 2 300 808
- DATABASE WPI Week 48571, 23 August 2019 Derwent World Patents Index; AN 2019-48571T

## Description

### OBJECT OF THE INVENTION

The present invention falls within the technical field of bandages or dressings, as well as that of devices for draining wounds or the like and relates in particular to a protective dressing for abdominal viscera in open abdomen for use in negative pressure therapy.

### BACKGROUND OF THE INVENTION

In the cases of surgical operations inside the abdominal cavity that require making transcutaneous incisions to access said interior, the primary closure of the abdominal cavity restores the anatomy and physiology of the abdominal wall, providing protection to the abdominal content and reducing the complications of the surgical wound. However, there are certain circumstances, such as abdominal trauma, severe acute pancreatitis, severe septic abdomen or Abdominal Compartment Syndrome, wherein primary closure is not possible, making it necessary to establish a system for wound management that facilitates reentry into the abdominal cavity and allow successive operations.

Forced closure or tension of the cavity can cause increased intra-abdominal pressure (IAP) in the patient and lead to the aforementioned Abdominal Compartment Syndrome (ACS), which is a condition that involves an increase in pressure in a compartment, in this case the abdominal cavity, which causes an anomalous venous return as well as a difficulty in the blood supply to the viscera, leading to hypo-perfusion and finally, with an increase in IAP, to ischemia due to a decrease in blood flow, arterial blood perfusion and venous congestion that can lead to the death of the patient.

Over the years, various strategies have been developed to try to mitigate intra-abdominal hypertension and prevent its progression to ACS. In addition to being necessary to facilitate reentry into the abdominal cavity, it may be desirable to remove fluids from the cavity and apply therapy that reduces pressure on the tissue or wound. This therapy, frequently referred to in the medical community as negative pressure therapy (NPT), provides a number of benefits: decreases wound retraction, removes exudate and non-viable tissue, improves blood supply, promotes granulation tissue generation, physically stimulates mitosis and, as a consequence of all this, facilitates healing and closure of the abdominal cavity.

Various devices for applying NPT as well as dressings and drainage components of said systems are known in the current state of the art. For example, the document under publication number WO2013034262 describes a dressing for negative pressure therapy comprising a flexible film, with a first and a second side, the first side being provided for application on the wound and, the flexible film comprising openings that are distributed over the entire surface.

Furthermore, said dressing comprises at least three sections of conduits, on the second side of said flexible film.

Although it is described that said film consists of a material that should have atraumatic properties, to prevent it from adhering to internal organs, said dressing generates a negative pressure on the mesothelium that passes through the dressing and, when withdrawn, produces a lifting of part of the tissue also leaving a mark on the tissues through the openings.

On the other hand, the patent application AU2016277595, describes a system for applying NPT that comprises a device for treatment with a plurality of members encapsulated and placed on a surface. Said members are coupled to a central connector. Fenestrations can be formed in the plurality of encapsulated members, or in the central connector, which allow fluids in the abdominal cavity to pass through. However, the encapsulated member quickly saturates and ceases to exert an effective negative pressure during therapy.

Document GB2300808A discloses a surgical dressing or bandage comprising a plastic material in which sealed pockets or bubbles are filled with gas or air. In addition, the sheet may contain holes or perforations between the pockets to allow the skin to breath. Preferably, the sheet has a layer of gauze and may also contain adhesive tape. The device provides cushioning protection and may be used in, but is not limited to, the management of bed sores and orthopedic injuries.

In view of the state of the art, there arises a need for devices that improve negative pressure therapy, facilitating the closure of the abdominal cavity and avoiding or minimizing complications, such as deep tissue retraction or necrosis.

### DESCRIPTION OF THE INVENTION

The object of the invention consists of a protective dressing for abdominal viscera for the application of negative pressure therapies in the open abdomen, which avoids the aforementioned drawbacks and also allows optimizing the distribution of negative pressure over the surface of the sheet to the most distal areas and improve drainage capacity.

To this end, said dressing comprises a plurality of laminar layers overlapped and integrally linked to each other by at least their respective perimeter edges. In its preferred embodiment, the dressing is made up of three layers: an upper layer, a lower layer and an intermediate layer, arranged between the upper and lower layers.

The upper and lower layers each consist of a laminar body with an internal side, an external side and a plurality of first through holes having reduced dimensions and uniformly distributed that allow the passage of fluids through them.

For its part, the intermediate layer consists of a laminar body that incorporates a plurality of alveoli or bubbles evenly distributed and filled with low pressure air. Low pressure air prevents possible damage to the abdomen. In a preferred embodiment, said bubbles have an essentially hemispherical geometry and are uniformly distributed throughout the intermediate layer.

It is also envisaged that said distribution may correspond to radial, concentric, spiral or barred patterns, as well as that the bubbles have a conical or cylindrical geometry. In alternative embodiments, the bubbles can be filled with CO₂ or other low-pressure gases.

This intermediate layer also incorporates a plurality of second through holes adjacent to the bubbles, to allow the passage of fluids through them, giving rise to an intermediate layer of a fenestrated nature. These second holes also allow the transmission of negative pressure to the abdominal cavity. In a preferred embodiment, the second through holes have an essentially rhomboid geometry.

The protective dressing for abdominal viscera thus described is temporarily placed on an open abdomen, in particular on the internal organs of the abdomen or on the greater omentum, indistinctly, on one of the external sides of the upper or lower layers. A spongy body is in turn arranged on the dressing, on which a negative pressure therapy system is supported.

By applying negative pressure to the dressing, the air-filled bubbles prevent the sheets from adhering to each other in the first place, as this configuration prevents rapid saturation and clogging of the polyurethane sponge contained in other dressings with more dense and solid elements like the remains of pus, fibrin clots and necrosis.

The bubbles also prevent adherence to the tissues of the internal organs of the abdominal wall, as well as the effluents from the internal organs (fluids, necrotic tissues, clots, etc.) drain homogeneously through the through holes. The uniform distribution of both the air bubbles and the through holes over the entire surface of the respective layers optimizes the drainage capacity and the distribution of negative pressure therapy, allowing it to be exercised even in the most distal areas of the cavity.

The dressing is preferably made of a material selected from the group of EVA, PU, PVC, PE, PET, PTFE, TPE, silicone or a mixture thereof. Also, the dressing can be adjusted to the size of the opening of the abdomen, depending on the treatment required, cutting the necessary surface.

### DESCRIPTION OF THE DRAWINGS

To complement the description that is being made and in order to help a better understanding of the features of the invention, according to a preferred example of a practical embodiment thereof, a set of drawings is attached as an integral part of said description in which, for illustrative and non-limiting purposes, the following has been represented:
Figure 1.- Shows an exploded perspective view of the dressing, wherein its main constituent elements are seen.
Figure 2.- Shows a plan view of the dressing.

### PREFERRED EMBODIMENT OF THE INVENTION

A detailed explanation of a preferred embodiment of the object of the present invention is provided below, with the aid of the aforementioned figures.

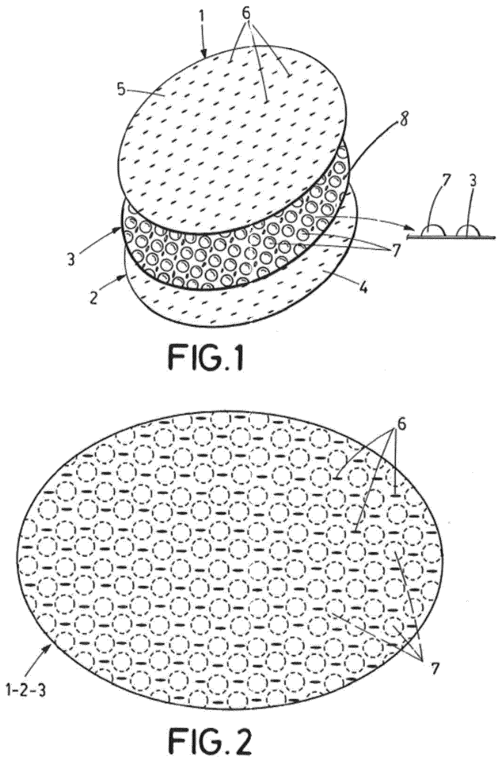

The protective dressing for abdominal viscera in negative pressure therapy that is described is made up of a multilayer structure, which in this preferred embodiment is comprised of an upper layer (1), a lower layer (2) and an intermediate layer (3) located between the upper (1) and lower (2) layers, the three layers being integrally linked to each other by their respective perimeter edges as seen in figure 1.

The upper (1) and lower (2) layers, having similar geometry and dimensions, each consist of a laminar body with an internal side (4), intended to be oriented inwards and facing the intermediate layer (3) and an external face (5), intended to be oriented towards the outside. Likewise, the laminar body incorporates a plurality of first evenly distributed through holes (6) that allow the passage of fluids through them, which gives said upper (1) and lower (2) layers a fenestrated nature.

The intermediate layer (3), having geometry and dimensions essentially similar to those of the upper (1) and lower (2) layers, consists of a laminar body that incorporates a plurality of bubbles (7) uniformly distributed and filled with a gaseous fluid at low pressure. In the preferred embodiment described herein, said bubbles (7) have an essentially hemispherical geometry, as illustrated in the detail in figure 1.

The intermediate layer (3) also incorporates a plurality of second through holes (8) adjacent to the bubbles (7) that allow the passage of fluids through them, giving rise to an intermediate layer (3) of a fenestrated nature, as well as allowing the transmission of negative pressure to the abdominal cavity. In this preferred embodiment, the second through holes (8) have an essentially rhomboid geometry. The separation distance between bubbles (7) in said intermediate layer (3) shall be at least half their diameter.

As can be seen in Figure 2, in the preferred embodiment the overlapping of the upper (1), lower (2) and intermediate (3) layers is carried out in such a way that the respective through holes (6) are facing each other, without the bubbles (7) being facing said through holes (6).

## Claims

1. Protective dressing for abdominal viscera for application of negative pressure therapy in open abdomen, wherein the dressing comprises a plurality of laminar layers overlapped and integrally linked to each other by at least their respective perimeter edges and wherein each of the layers consists of a laminar body, being the protective dressing **characterized in that** it consists of:
- an upper layer (1) which includes a plurality of first through holes (6) for the passage of fluids through them;
- an intermediate layer (3) which includes:
- a plurality of bubbles (7) filled with a low-pressure gaseous fluid, and
- a plurality of second through holes (8) for the passage of fluids through them; and
- a lower layer (2) that includes the first through holes (6) for the passage of fluids through them;
- wherein the layers (1, 2, 3) overlap each other so that the first through holes (6) face each other with interposition of the second through holes (8) of the intermediate layer (3) without the bubbles (7) being facing said through holes (6) for improving drainage capacity; and
- wherein the intermediate layer (3) is of a fenestrated nature for optimizing the transmission of negative pressure to the abdominal cavity.

2. Protective dressing according to claim 1, wherein two contiguous bubbles (7) are separated from each other by a distance of at least half their diameter.

3. Protective dressing according to any of the preceding claims, wherein the bubbles (7) have an essentially hemispherical geometry.

4. Protective dressing according to any of the preceding claims, wherein the bubbles (7) are filled with CO₂.

5. Protective dressing according to any of the preceding claims, wherein it is made of a material selected from the group comprising EVA, PU, PVC, PE, PET, PTFE, TPE, silicone or a mixture thereof.

## Patentansprüche

1. Schutzverband für Baucheingeweide für die Anwendung einer Unterdrucktherapie in einem offenen Abdomen, wobei der Verband eine Vielzahl von Laminarschichten umfasst, die einander überlappen und durch zumindest ihre Umfangsränder fest miteinander verbunden sind, und wobei jede der Schichten aus einem Laminarkörper besteht, wobei der Schutzverband **dadurch gekennzeichnet ist, dass** er aus Folgendem besteht:
- einer oberen Schicht (1), die eine Vielzahl von ersten Durchgangslöchern (6) zum Durchtritt von Fluid durch diese hindurch umfasst;
- einer Zwischenschicht (3), die Folgendes umfasst:
- eine Vielzahl von Blasen (7), die mit einem Niederdruckgasfluid gefüllt sind, und
- eine Vielzahl von zweiten Durchgangslöchern (8) zum Durchtritt von Fluid durch diese hindurch;
und
- einer unteren Schicht (2), die die ersten Durchgangslöcher (6) zum Durchtritt von Fluid durch diese hindurch umfasst;
- wobei die Schichten (1, 2, 3) einander überlappen, derart, dass die ersten Durchgangslöcher (6) unter Zwischenanordnung der zweiten Durchgangslöcher (8) der Zwischenschicht (3) einander gegenüberliegen, ohne dass die Blasen (7) den Durchgangslöchern (6) gegenüberliegen, um die Drainagefähigkeit zu verbessern; und
- wobei die Zwischenschicht (3) eine fenestrierte Beschaffenheit zum Optimieren der Übertragung von Unterdruck in die Bauchhöhle aufweist.

2. Schutzverband nach Anspruch 1, wobei zwei aneinandergrenzende Blasen (7) um einen Abstand von mindestens der Hälfte ihres Durchmessers voneinander getrennt sind.

3. Schutzverband nach einem der vorangehenden Ansprüche, wobei die Blasen (7) eine im Wesentlichen halbkugelförmige Geometrie aufweisen.

4. Schutzverband nach einem der vorangehenden Ansprüche, wobei die Blasen (7) mit CO₂ gefüllt sind.

5. Schutzverband nach einem der vorangehenden Ansprüche, wobei dieser aus einem Material hergestellt ist, das aus der Gruppe ausgewählt ist, die EVA, PU, PVC, PE, PET, PTFE, TPE, Silikon oder ein Gemisch davon umfasst.

## Revendications

1. Pansement protecteur des viscères abdominaux pour l'application d'une thérapie par pression négative en abdomen ouvert, le pansement comprenant une pluralité de couches lamellaires superposées et reliées entre elles de manière solidaire par au moins leurs bords périphériques respectifs et dans lequel chacune des couches est constituée d'un corps laminaire, le pansement étant **caractérisé en ce qu'**il consiste en :
- une couche supérieure (1) comportant une pluralité de premiers trous traversants (6) pour le passage de fluides à travers eux ;
- une couche intermédiaire (3) qui comporte :
- une pluralité de bulles (7) remplies d'un fluide gazeux à basse pression, et
- une pluralité de deuxièmes trous traversants (8) pour le passage de fluides à travers eux ; et
- une couche inférieure (2) qui comporte les premiers trous traversants (6) pour le passage de fluides à travers eux ;
- dans lequel les couches (1, 2, 3) se chevauchent de sorte que les premiers trous traversants (6) se font face, avec interposition des seconds trous traversants (8) de la couche intermédiaire (3), sans que les bulles (7) ne soient en face desdits trous traversants (6), afin d'améliorer la capacité de drainage ; et
- dans lequel la couche intermédiaire (3) est fenêtrée afin d'optimiser la transmission de la pression négative à la cavité abdominale.

2. Pansement protecteur selon la revendication 1, dans lequel deux bulles contiguës (7) sont séparées l'une de l'autre d'une distance d'au moins la moitié de leur diamètre.

3. Pansement protecteur selon l'une quelconque des revendications précédentes, dans lequel les bulles (7) ont une géométrie essentiellement hémisphérique.

4. Pansement protecteur selon l'une quelconque des revendications précédentes, dans lequel les bulles (7) sont remplies de CO₂.

5. Pansement protecteur selon l'une quelconque des revendications précédentes, constitué d'un matériau choisi dans le groupe comprenant l'EVA, le PU, le PVC, le PE, le PET, le PTFE, le TPE, le silicone ou un mélange de ceux-ci.
